# EUROPÄISCHE PATENTSCHRIFT

(11) **EP 1 833 436 B1**
(45) Veröffentlichungstag und Bekanntmachung des Hinweises auf die Patenterteilung: **29.08.2012**
(21) Anmeldenummer: 06700107.3
(22) Anmeldetag: 04.01.2006
(51) Int. Cl.: A61F 5/56, A61F 2/00

(54) **IMPLANTIERBARE VORRICHTUNG ZUR NICHT PNEUMATISCHEN, JUSTIERBAREN POSITIONIERUNG EINES MENSCHLICHEN ODER TIERISCHEN KÖRPERTEILS**
IMPLANT DEVICE FOR THE NON-PNEUMATIC ADJUSTABLE POSITIONING OF A HUMAN OR ANIMAL BODY PART
DISPOSITIF IMPLANTABLE PERMETTANT LE POSITIONNEMENT AJUSTABLE, NON PNEUMATIQUE, D'UNE PARTIE D'UN CORPS HUMAIN OU ANIMAL

(30) Priorität: 04.01.2005 DE 102005000702
(43) Veröffentlichungstag der Anmeldung: 19.09.2007
(73) Patentinhaber: Klinikum der Universität Regensburg, 93053 Regensburg (DE)
(72) Erfinder: KÜHNEL, Thomas, 93053 Regensburg (DE)
(74) Vertreter: Vossius & Partner
(86) Internationale Anmeldenummer: PCT/EP2006/000031
(87) Internationale Veröffentlichungsnummer: WO 2006/072571

(56) Entgegenhaltungen:
- WO-A-2004/021869
- WO-A-2004/084709
- DE-C1- 19 756 956
- US-A1- 2004 139 975
- US-A1- 2004 149 290
- US-A1- 2005 092 334

## Beschreibung

Die Erfindung betrifft eine implantierbare Vorrichtung zur nicht pneumatischen, justierbaren Positionierung eines menschlichen oder tierischen Körperteils. Insbesondere betrifft die Erfindung eine Vorrichtung zur Verwendung zur nicht pneumatischen, justierbaren Positionierung eines Zungenkörpers. Die Verwendung der Vorrichtung erfolgt bevorzugt durch Implantation der Vorrichtung im entsprechenden Gewebe. Durch Verwendung der Vorrichtung lassen sich insbesondere schlafbezogene obstruktive Atmungsstörungen, wie zum Beispiel die Schlafapnoe, erfolgreich behandeln.

Schlafbezogene obstruktive Atmungsstörungen sind in den westlichen Industrienationen außerordentlich häufig. Schätzungsweise 9% aller Menschen sind von einer derartigen Störung betroffen. Die Deutsche Gesellschaft für Schlafmedizin und Biochronologie schätzt die Zahl betroffener Männer in Deutschland auf 200.000. Die Kosten für die Behandlung der Erkrankten in Deutschland betragen geschätzte € 700.000.000.

In einem schlafenden Organismus erfährt die Muskulatur einen Teilverlust ihrer Spannung. Dies führt im Bereich der Atemwege oberhalb des Kehlkopfs zur relativen Instabilität der Atemwege, da hier keine Skelettanteile wie Knorpel oder Knochen die Atemwege offen halten. Die Zunge bewegt sich in Rückenlage des Patienten bei nachlassender Muskelspannkraft der Schwerkraft und aerodynamischen Kräften folgend auf die Rachenhinterwand zu und engt somit die Atemwege auf Niveau des Zungengrundes, des Hypopharynx und des Velums ein: Bei schlafgesunden Menschen wirken verschiedene Mechanismen, u.a. eine angepasste nervale Regelung der Muskulatur einem Kollaps entgegen. Bei circa 2 bis 4% der Menschen in den Industrieländern kommt es jedoch während des Schlafes durch eine Störung dieser Regelung zu wiederholten (Teil-)Verschlüssen des Atemrohres mit nachfolgender Störung des Schlafes durch Sauerstoffverarmung des Blutes und/oder Weckreaktionen (= Arousels). Eine Kaskade von Folgereaktionen stört den Schlaf und dessen Erholungsfunktion. Leitsymptom dieser Störung ist exzessive Tagesmüdigkeit.

Gegenwärtig werden schlafbezogene obstruktive Atmungsstörungen durch eine apparative pneumatische Schienung der Atemwege durch von außen mittels Kompressor zugeführte Pressluft über eine Nasenmaske (nCPAP) behandelt. Dieses Verfahren hat sich in der Routine bewährt und gilt derzeit als Goldstandard der Apnoe-Behandlung. Nachteilig ist der wenig komfortable Schlaf durch das allnächtlich zu tragende Gerät verbunden mit Problemen, die an den Schleimhäuten auftreten können sowie zum Teil einer schlechten Akzeptanz insbesondere bei jüngeren Patienten. Alternativ wird das Leiden operativ behandelt. Keines der bekannten Verfahren ist jedoch risikofrei und in der Wirkung sicher.

Ein üblicher Ansatz ist die Reduktion von als überschüssig bewertetem Weichteilgewebe oder die mehr oder weniger gezielt provozierte Narbenbildung zur Stabilisierung des Weichgaumens. Verschiedene Operationsverfahren stehen hierbei zur Verfügung. So werden bei dem Radiofrequenzablationsverfahren mit Wechselstrom im Zungengewebe und dem Weichgaumen thermische Läsionen erzeugt, die zu Narben führen, was wiederum zu einer Gewebeschrumpfung mit Versteifung des umliegenden Gewebes führt. Im Bereich der Atemwege zwischen Nasenrachen und Kehlkopf fehlt die passive Stütze von Knochen oder Knorpel, die einen Kollaps verhindern könnte. Die Luftröhre z.B. wird durch Knorpelspangen stabilisiert. Sie sind auf der Rückseite offen, in Grenzen elastisch und durch eine bindegewebige Membran verbunden. Bei der Uvulopalatopharyngoplastik und der laserassistierten Uvulopalatoplastik (UPPP/LAUP) wird durch Schnitte im Weichgaumen eine Erweiterung der Rachenenge und eine Versteifung des schwingungsfähigen Weichgewebes erzielt. Ein anderes operatives Prinzip zielt auf die Korrektur oder Veränderung anatomischer Strukturen. Hierzu gehört die Verlagerung von Muskelansätzen, die Fixierung des Zungenbeins am Kehlkopf und die Vorverlagerung des Unterkiefers bzw. des gesamten Mittelgesichts. Bei der Hyoidfixation wird das Zungenbein mit mäßigem Zug gegen den Kehlkopf fixiert. Bei der Unterkiefervorverlagerung erfolgt ein kieferchirurgischer Eingriff bei dem der Unterkiefer zersägt wird und mit Metallplatten nach vorne verlagert wird. Das entsprechend Operationsverfahren ist aufwendig und nicht risikofrei. Bei dein bimaxillärem Advancement werden Ober- und Unterkiefer zersägt und das gesamte Mittelgesicht vorverlagert. Trotz eines meist guten Ergebnisses haben der sehr große operative Aufwand und das Risiko kosmetischer Nachteile eine Verbreitung der Methode verhindert. Bei der Tracheotomie erfolgt ein Luftröhrenschnitt zur Umgehung der oberen Atemwege. Aktuell sind jedoch operative Verfahren zu wenig effektiv und/oder zu belastend für den Patienten.

Ferner ist ein medizinisches Gerät zur Implantation unter der Marke ₙRepose" von der israelischen, jetzt US-amerikanischen Firma Influ∼ENT auf dem Markt. Hierbei wird ein nicht resorbierbarer Faden unter der Schleimhaut durch den Zungenkörper geführt und an der Innenseite des Unterkiefers mit einer Titanschraube fixiert. Die selbstschneidende Titanschraube wird nach Durchtrennen der Schleimhaut mit einem Winkelakkuschrauber eingedreht. Dadurch wird ein Kontakt der Zunge mit der Rachenhinterwand während des Schlafes verhindert. Dieses System, das in der EP 0 998 224 beschrieben ist, hat sich jedoch in der Praxis in verschiedenen Aspekten als nachteilig erwiesen. So liegt nach der Operation eine nicht korrigierbare Fadenspannung vor. Die richtige Fadenlänge kann während der Operation nur abgeschätzt werden, wobei es jedoch keine konkreten Anhaltspunkte für die zu wählende Fadenlänge gibt. Ferner ist das System nicht elastisch und wird daher der in hohem Maße veränderlichen Zungenform nicht gerecht. Der Faden kann nach der vom Hersteller angegebenen Methode auch nicht reproduzierbar geradlinig verlegt werden. Äußerst nachteilig ist, dass die Fadenspannung sich intraoperativ nicht bedarfsorientiert dosieren lässt. Ein sinnvolles Maß für die Fadenspannung ist durch eigene Untersuchungen nicht zu ermitteln gewesen. Fer ner komprimiert die Fadenschlinge das Gewebe und die darin verlaufenden Blutgefäße und Nerven. Dies wird zusätzlich durch Bewegungen der Zunge, insbesondere den Schluckakt, verstärkt, wobei der Faden stark gespannt wird. Zumindest während der Phase der Wundheilung durchwandert der Faden in unterschiedlichem, nicht kalkulierbarem Umfang das Zungengewebe. Weiterhin besteht die Gefahr von Komplikationen durch die Nähe der Titanschraube zu den Zahnwurzeln. Es besteht Verletzungsgefahr der Speicheldrüsenausführungsgänge. Ferner ist die Vorrichtung auch nicht geeignet, andere Körperteile wirksam in Position zu halten.

Die Druckschriften WO 99/32057 A1, US 2004/149290 A1, WO 2004/21869 A2, US 2004/139975 A1, WO 2004/084709 A2, WO 2004/32798 A1, US 4,978,323 A, DE 199 20 114 A1 und WO 99/58 A1 beschreiben Vorrichtungen, um die Atemwege freizuhalten. Diese Vorrichtungen besitzen jedoch unter Anderem den Nachteil, dass sie prä- und postoperativ nicht mehr justiert werden können und meist anatomisch bedeutsame Strukturen gefährden.

Zudem wird auf die Druckschriften US 2004/139975 A1, DE 197 56 956 C1, US 2004/149290 A1, WO 2004/021869 A und WO 2004/084709 A verwiesen.

Der vorliegenden Erfindung liegt somit die Aufgabe zugrunde, eine Vorrichtung bereitzustellen, durch die menschliche oder tierische Körperteile einfach, wirksam und justierbar positioniert werden können und somit die durch fehlerhafte bzw. mangelnde Positionierung der entsprechenden Körperteile hervorgerufenen Störungen wirksam und ursächlich und dauerhaft behandelt werden können. Die durch die Vorrichtung erzeugte Repositionierung soll einfach vorzunehmen und nebenwirkungsarm sein.

Der vorliegenden Erfindung liegt insbesondere die Aufgabe zugrunde, eine Vorrichtung bereitzustellen, mit der durch mangelnde Positionierung des Zungenkörpers hervorgerufene Störungen, insbesondere schlafbezogene, obstruktive Atmungsstörungen wirksam, nebenwirkungsarm und einfach behandelt werden können. Durch die Vorrichtung soll die Zunge in ihrer Beweglichkeit nicht oder nur kaum eingeschränkt werden und es sollen Schlucken und Sprechen nicht behindert werden. Ferner soll das Gerät eine einfach vorzunehmende Justierung ermöglichen, um den sich ändernden Anforderungen seitens der Erkrankung bzw. Störung gerecht zu werden. Das Gerät soll ferner technisch einfach zu implantieren sein. Vorzugsweise soll das Gerät mit handelsüblichem Routineinstrumentarium eines HNO-Chirurgen implantiert werden können.

Diese Aufgabe wird durch die Merkmale des unabhängigen Anspruchs gelöst. Bevorzugte erfindungsgemäße Ausführungen sind in den abhängigen Ansprüchen definiert.

Insbesondere wird diese Aufgabe gelöst durch eine implantierbare Vorrichtung zur nicht pneumatischen, justierbaren Positionierung eines menschlichen oder tierischen Körperteils umfassend a) mindestens ein elastisches Element und gegebenenfalls, b) ein Widerlager, sowie durch die Verwendung der Vorrichtung in einem Verfahren zur Behandlung von Störungen, die durch fehlerhafte oder mangelnde Positionierung eines menschlichen oder tierischen Körperteils hervorgerufen werden, insbesondere zur Behandlung von Atmungsstörungen, schlafbezogenen Atmungsstörungen, obstruktivem Schnarchen, Glossoptose und ähnlichen Erkrankungen.

Überraschenderweise wurde gefunden, dass ein System, bei dem mindestens ein elastisches Element vorliegt, sich vorteilhafterweise zur nicht pneumatischen, justierbaren Positionierung eines menschlichen oder tierischen Körperteils eignet. Das System enthält gegebenenfalls noch ein Widerlager.

Sofern das System nur aus einem elastischen Element besteht, kann dieses in Form einer Spange oder eines Rings vorliegen. Das elastische Element kann ferner ein oder mehrteilig sein und liegt in diesem Fall zusammen mit einem Widerlager vor.

Die elastischen Eigenschaften der erfindungsgemäßen Vorrichtung sollen denen des umgebenden Gewebes nahe kommen. Um die Wahrscheinlichkeit einer Extrusion zu verringern und dennoch die gewünschte Gegenkraft zum kritischen Kollapsdruck und der Gewichtskraft erzielen zu können muss die erfindungsgemäße Vorrichtung ein elastisches Element enthalten, das reversibel in dem zu positionierenden Körperteil verankert wird. Das elastische Element erlaubt die Justierung der erforderlichen Kraft in die gewünschte Richtung. Durch verschieden starkes Dehnen lässt sich die Kraft verändern. Dadurch kann auch eine beim Ersteingriff nicht optimal eingestellte Spannung zu einem späteren Zeitpunkt korrigiert werden. Die Verbindung des elastischen Elements mit weiteren Elementen der erfindungsgemäßen Vorrichtung kann durch Verriegeln oder Verkleben erfolgen.

Das elastische Element erlaubt eine diskrete Anpassung der implantierbaren Vorrichtung an die jeweiligen Gegegebenheiten des Organismus, in den das lmplantat-eingebracht werden soll.

Die Vorspannung des elastischen Elements lässt sich durch einen kleinen Eingriff in örtlicher Betäubung verändern, wobei das elastische Modul des Geräts gegebenenfalls ausgewechselt werden kann. Bevorzugt wird dabei die Vorspannung des elastischen Elements als solches (bei einteiliger Vorrichtung) bzw. die Positionierung des elastischen Elements in Bezug auf das Widerlager geändert. Die Vorrichtung kann über einen Zugang von außen, also über einen Hautschnitt, implantiert werden. Das elastische Element erlaubt eine stufenlose bzw. schrittweise Anpassung des Systems an die unterschiedlichen anatomischen Gegebenheiten des Patienten sowie an die im Laufe des Lebens wechselnden Erfordernisse des Patienten. Durch die Vorrichtung wird eine Kraft entgegen der Schwerkraft und gegen den kritischen Kollapsdruck ausgeübt, die größer ist als beispielsweise die Gewichtskraft der Zunge aber kleiner als die durch die Zunge selbst entwickelte Muskelkraft beim Sprechen oder Schlucken ist, bzw. entsprechender Kräfte bei der Positionierung anderer Körperteile. Weiterhin kann die erfindungsgemäße Vorrichtung zur Behandlung von Atmungsstörungen von außen, also submental, implantiert werden, während die diesbezügliche Vorrichtung des Stands der Technik (Repose) enoral, also über die Zähne hinweg zum Mundboden implantiert wird.

Die Anordnung der Komponenten im elastischen Element kann dergestalt sein, dass in sagittaler Richtung bewegliche Teile des Mundbodens und der Zunge die elastischen Komponenten aufnehmen und die in sagittaler Richtung weniger beweglichen anatomischen Strukturen die rigiden oder geringer elastische Teile des Elements aufnehmen. Bevorzugt ist der elastischere Teil des Bandes dem Anker benachbart, ein rigiderer Anteil des Bandes dem Widerlager. Die Verbindung der Komponenten kann durch Klebung erfolgen oder durch Einbringen eines Materials kleinerer Elastizität in das elastische Element (Armierung).

Sofern die erfindungsgemäße Vorrichtung nur aus einem elastischen Element besteht, liegt dieses bevorzugt in ringförmiger Form vor und ist besonders bevorzugt zur Implantation im Pharynx vorgesehen. Es erweist sich als besonders vorteilhaft zur Behandlung schlafbezogener Atmungsstörungen. Das Atemrohr ist im Kollapssegment nicht wie an anderen Stellen (Luftröhre, Kehlkopf, Nase) durch Knorpel oder Knochen gestützt. Die Muskelspannung garantiert beim Wachen und Schlafgesunden die Öffnung des Kollapssegments. Ein Kranker besitzt keine ausreichende Muskelspannung, so dass der atmosphärische Druck das Atemrohr zusammenpresst. Unterschiedliche Kollapsformen werden beobachtet. Dabei sinkt vor allem die Zunge nach hinten. Ein zirkuläres Implantat besitzt den Vorteil, dass es dem kritischen Kollapsdruck widersteht und aber durch seine Elastizität den gewollten Kollaps des wachen Menschen ermöglicht. Ein derartiges kreisförmiges Implantat kann aus mehreren schmalen zirkulären Implantaten bestehen oder kann aus einem Rundkörper bestehen. Dieser besteht aus einem biokompatiblen Material, vorzugsweise Kunststoff, z.B. Silikon.

Die Implantation einer ringförmigen Vorrichtung erfolgt zweckmäßigerweise durch Legen einer Pfandfindersonde (kräftiger Faden) und anschließendes Durchziehen des Implantats unter der Schleimhaut oder der oberflächlichen Muskelschicht. Die physiologische Hauptbewegung (Schlucken, Sprechen) ist eine Annäherung des Zungengrunds an die Rachenhinterwand. Daher muss das Implantat an den Seiten eine Sollknickstelle besitzen. Dies wird durch eine Stelle mit besonders schwachem Materialeinsatz erzielt. Die Dimensionierung der Spange, die in einer bevorzugten Ausführungsform geschlossen sein kann und dann als Ring vorliegt, hängt im Wesentlichen von drei Faktoren ab. Hierbei wird auf die Implantation im Rachengewebe Bezug genommen. Die Faktoren sind die gewünschte Rückstellkraft, die individuelle Konfiguration des Schlundes und die Menge an Gewebe, das von der Spange am Kollaps gehindert werden soll. Hierbei wird die Spange in Bezug zur Wirbelsäule gesetzt. Im Bereich der Wirbelsäule ist die Spange offen bzw. weist die geringste Materialstärke auf. Seitlich besitzt die Spange idealerweise Sollknickstellen, um die Rückwärtsbewegung der Zunge beim Schlucken und Sprechen zuzulassen. Die elastische Rückstellkraft ist gegengleich dem pharyngealen Verschlussdruck während einer Apnoe oder knapp darüber. In Richtung Zungengrund ist die Spange breiter, um ausreichend Gewebe zu erfassen. Nachstehend ist ein Beispiel für eine mögliche Dimensionierung einer Spange aus dauerimplantierbarem Silikon Nusil Shorhärte 75 gegeben:

| | |
|---|---|
| Maximale Breite: | 15 mm |
| Minimale Breite | 1 mm |
| Länge: | 75 mm |
| maximale Materialstärke: | 2 mm |
| minimale Materialstärke: | 0,5 mm |
| Öffnung der Spange: | 20 mm. |

Durch Ausdünnen des Silikons zu den Spangenenden hin wird die Rückstellkraft des Implantats an dieser Stelle so verringert, bis sie annähernd den elastischen Eigenschaften des Gewebes entspricht und sich damit die Extrusionswahrscheinlichkeit verringert. Zur alleinigen Implantation ist das elastische Element geeignet, wenn seine elastische Rückstellkraft ausreichend groß ist, einem krankhaften Kotlapsdruck, der vorwiegend nach hinten und von der Seite zur Mitte hin wirkt, zu widerstehen. Ein ringförmiges Implantat hat bei der Behandlung schlafbezogener Atmungsstörungen den Vorteil, dass die aufgenommenen Kräfte, die beim Schluckakt ihre Maximalwerte erreichen, nicht unmittelbar in das Gewebe abgeleitet werden, sondern vom rückseitigen Anteil des Implantats aufgenommen werden.

Die erfindungsgemäße Vorrichtung kann auch mehrteilig vorliegen. Ein mehrteiliges Implantat ist charakterisiert durch mindestens einen verformbaren beweglichen und einen nicht beweglichen Bauteil. Der nicht bewegliche Bauteil dient als Widerlager und stellt die Verbindung zu einem Referenzpunkt, zum Beispiel einer knöchernen Struktur wie dem Unterkiefer, dar. Der verformbare bewegliche Anteil des Implantats wird als elastisches Element bezeichnet. Dieses elastische Element kann seinerseits ein- oder mehrteilig sein. Das elastische Element liegt in Form eines Ankers und eines Bandes vor. Diese soll nun im Folgenden näher beschrieben werden.

Das elastische Element kann beliebige Formen annehmen und liegt bevorzugt bandförmig vor. Geeignet sind hierfür alle Materialien, die das Kriterium der Dauerelastizität erfüllen, beispielsweise Silikon, Verbundwerkstoffe, Stahlfedern. Ein fadenförmiges elastisches Element, auch Ankerfaden genannt, kann als Band oder Rundkörper ausgebildet sein. Das fadenförmige Element kann für radiologische Verfahren durch Einschlüsse verschiedener kontrastgebender Mittel, wie zum Beispiel Bariumsulfat, kontrastgebend gemacht werden, solange die Materialeigenschaften und die Biokompatibilität nicht negativ beeinflusst werden. Bevorzugt ist das elastische Element mit einer Umhüllung versehen. Diese Umhüllung stellt die Grenzschicht gegen den Körperteil dar und kann z.B. aus Polyurethan, Silikonfolie oder Polyethylen sein. Das elastische Element kann sich so in den erforderlichen Grenzen bewegen ohne durch Narben gehindert zu werden. Die Umhüllung soll nicht resorbierbar sein, die Integration in das Gewebe fördern und bakteriostatisch/bakterizid sein. Liegt das elastische Element in einer Silikonausführung oder in einer entsprechenden Materialausführung vor, ist eine Ummantelung nicht erforderlich.

Bei Silikonausführung kann durch eine besonders glatte Oberfläche die Integration in das Gewebe verhindert werden und die Beweglichkeit relativ zur Umgebung erhalten werden. Dies ist z.B. durch das Funkenerosionsverfahren bei der Herstellung des Nestes zu erreichen. Das Silikon kann mit Silberionen dotiert werden (Nanosilber, Samuel U, Guggenbichler JP: Prevention of catheter-related infections: the potential of a new nano-silver impregnated catheter. Int J. Antimicrob Agents, 2004 Mar; 23 Suppl. 1:S75-8..) um eine bakteriziden/bakteriostatischen Effekt zu erzielen.

Das bandförmige elastische Element kann verschieden ausgeführt sein. Beispielsweise kann es als Polyeder (zum Beispiel als Quader), als Rotationskörper oder als Teil eines Rotationskörpers ausgeführt sein. Ferner kann es an den Kanten abgerundet sein. Weiterhin kann das Band an einer oder mehreren Seiten Vertiefungen oder Erhöhungen aufweisen. Diese können zum Beispiel Mulden, Löcher, Kerben oder Noppen sein. In einer weiteren Ausführungsform besitzt das Band ein- oder beidseitige, runde, ovale oder vieleckiger Mulden konischen oder zylindrischen Querschnitts zur Aufnahme eines durch den gesamten Querschnitt reichenden Verriegelungsbolzens/Schraube oder zur Aufnahme eines nur teilweise durch das Band reichenden Bolzens. In einer weiteren Ausführungsform besitzt das Band runde, ovale oder vieleckige durch den Bandquerschnitt reichende Öffnungen (Durchtrittslöcher) konischen oder zylindrischen Querschnitts. In diese kann ohne Weiteres ein Bolzen oder eine Schraube eingebracht werden.

In einer weiteren Ausführungsform besitzt das Band runde, ovale oder vieleckige durch den Bandquerschnitt reichende Öffnungen konischen oder zylindrischen Querschnitts. Durch eine dünne Materiallamelle in der Querschnittsmitte wird verhindert, dass Gewebe vollständig durch das Loch wächst. Diese Lamelle kann ohne Weiteres mit einem konischen Instrument (Durchschlag) entfernt werden. Die Lamelle unterschiedlicher Dicke kann die Öffnung einseitig verschließen. Der Verriegelungsbolzen kann komplett oder nur partiell durch das Band reichen.

In weiteren Ausführungsformen sind die Mulden/Öffnungen in einem engen Abstand angeordnet, so dass ein annähernd stufenloser Verstellmechanismus resultiert. Die Mulden bzw. Öffnungen können auch in einem größeren Abstand, zum Beispiel von 18 mm angeordnet sein, so dass ein Ausreißen des Bandes aus dem Gewebe unwahrscheinlich wird. Die Mulden oder Löcher können einen so kleinen Durchmesser aufweisen, dass sie keinen merklichen Einfluss auf die Stabilität des Bandes haben. Durch Dehnung wird der Durchtritt der Schraube/eines Bolzens ermöglicht.

Eine weitere Ausführungsform des bandförmigen elastischen Elements stellt ein elastisches Band mit Fischschuppenstruktur dar. Diese Ausführungsform kann aus dauerelastischem Material oder aber aus resorbierbarem Material gefertigt sein. In diesem letzteren Fall resultiert langfristig eine Narbe, die die gewünschten Eigenschaften auf das umliegende Gewebe vermittelt. Derartige Bänder sind im Handel erhältlich z.B. von der Firma Johnson u. Johnson. Diese besondere Oberflächenstruktur erlaubt eine Bewegung nach Implantation im Gewebe nur noch in eine Richtung. In einer Ausführungsform ist ein derartiges Band zum Beispiel am Zungengrund so breit, dass weitere Elemente der Vorrichtung entfallen können und dieses Band als alleiniges Implantat verwendet werden kann. In der für die Implantation im Mundbereich vorgesehenen Ausführungsform ist zum Mundboden. hin die Ausrichtung der Fischschuppenoberfläche gegengleich der Ausrichtung am Zungengrund. Das Fischschuppenband wird bevorzugt unter die harte Knochenhaut gezogen. An dieser Stelle ist eine ungewollte Bewegung nach dorsal praktisch nicht möglich, so dass sich im Effekt dadurch eine Kompression des Gewebes erzielen lässt.

Gemäß einer weiteren Ausführungsform besitzen das bzw. die elastischen Element/Elemente an ihrer Spitze, die im Gewebe verbleibt, Widerhaken. Diese Widerhaken verhindern die Dislokation des Implantats unter Zug.

Gemäß einer weiteren Ausführungsform besitzt das elastische Element eine Federkonstante, die durch eine spezifische Materialstärke erzielt wird. Dabei ist das elastische Element aus einem homogenen Material unterschiedlicher Materialstärke gefertigt. Bevorzugt besteht das elastische Element aus Silikon, die auf das Zungengewebe wirkende Kraft beträgt bei Dehnung um 10 mm 3N. Die Breite des elastischen Bandes ist ca. 4 mm. Ist bei einer bestimmten Patientenkonstellation eine kleinere Kraft erforderlich, kann ein Implantat mit kleinerem Durchmesser des elastischen Bandes angefertigt werden.

Gemäß einer weiteren Ausführungsform wird die Federkonstante des elastischen Elements durch einen spezifischen Materialtypus erzielt. Kleinere Rückstellkräfte werden durch Silikon kleinerer Shorhärte, größere durch Silikon höherer Shorhärte erzielt. Das elastische Element kann auch aus unterschiedlichen Materialien bestehen, zum Beispiel Silikon unterschiedlicher Härte oder aneinandergefügte Materialien, wie zum Beispiel Metalle, Kunststoff, textile Stoffe oder Verbundwerkstoff.

Gemäß einer weiteren Ausführungsform wird die gewünschte Federkonstante des elastischen Elements durch das Zusammenwirken verschiedener Komponenten erzielt. Es kann zum Beispiel eine Stahlfeder oder ein Faden in das elastische Element in ganzer Länge oder in Teilabschnitten eingebracht sein. Es entsteht so ein Verbund aus elastischen und weniger elastischen Komponenten. Weniger elastisch kann auch rigide bedeuten. Die Feinjustierung wird durch eine entsprechende Vordehnung bewerkstelligt. Es ist Aufgabe und Eigenschaft des elastischen Elementes, eine elastische Kraft mit definierter oder definierbarer Federkonstante bei Drehung durch die Zungenbewegung relativ zur Rachenhinterwand, zum Unter- und Oberkiefer und anderer Strukturen des Pharynx zu erreichen.

In einer Ausführungsform liegt das elastische Element in Form eines oder mehrer Schläuche vor. Zur Implantation ist ein Trokar erforderlich, der einen Kanal umschließt, durch den ein Draht in die Zielregion der Zunge vorgeschoben werden kann. Der Kanal weist an seinem Ende eine Krümmung auf, die den Draht im gewünschten Winkel entlässt. In einer Ausführungsform beträgt dieser Winkel 90°. Der Trokar besteht aus einer Hülle, einer Spitze, einer Auslassöffnung an der Seite und einer Einschuböffnung. An der Stelle der Auslassöffnung weist der Trokar den größten Durchmesser (zum Beispiel 10 mm) auf, um der Krümmung des Führungskanals Raum zu geben. Der Draht wird über den Trokar in sagittaler Richtung der Zunge von submental bis in den submukösen Raum vorgeschoben. Durch die Form des Trokarinneren wird er dann beim Verlassen des Trokars zur Seite abgelenkt. Der Draht dringt durch die Muskulatur bis zum seitlichen Rand der Zunge. Die auf diesem Weg in die Zunge eingebrachten Körper können in ihrer Elastizität nachträglich beeinflusst werden. Die Vorspannung des Schlauches wird verändert, indem ein Kern eingebracht wird. Dieser Kern kann aus beliebigem Material bestehen. In einer bevorzugten Ausführungsform besteht er aus Metall. Weitere geeignete Materialien sind Textilien oder Kunststoffe (zum Beispiel Polyurethan, Polytetrafluorethylen oder Thermoplasten).

In einer anderen Ausführungsform wird die Steifigkeit erhöht, indem das elastische Element gespannt wird. Der beschriebene Kern kann durch ein Gewinde, durch Verkleben oder einen anderen mechanischen, in der Technik üblichen, Mechanismus am zungenseitigen Ende des elastischen Schlauches befestigt werden. Am kinnseitigen Ende wird die Spannung der Konstruktion eingestellt. Möglich ist dies beispielsweise durch ein Gewinde auf dem Kern, über das eine Mutter geschraubt wird, die einen größeren Außendurchmesser als der elastische Schlauch aufweist. Durch Anziehen der Mutter wird der Kern relativ zum elastischen Schlauch verkürzt. Es entsteht eine Spannung zwischen Schlauch und Kern, die in erhöhter Steifigkeit resultiert. Die Steifigkeit des Kernes kann auch durch thermische oder chemische Verfahren erhöht werden.

Ein weiteres Element des Systems ist eine Ankervorrichtung bzw. ein Anker. Diese Ankervorrichtung kann beliebige Formen annehmen, wobei das grundlegende Prinzip ist, dass die Kraft auf einerseits eine möglichst große Gewebefläche übertragen wird, andererseits der Anker selbst eine kleine Oberfläche aufweisen soll. Dadurch sollen die Wahrscheinlichkeit einer Kolonisation bzw. Infektion des Fremdmaterials mit pathogenen Keimen und die Fremdkörperreaktion so klein wie möglich gehalten werden. Ein für die Funktion wichtiges Merkmal ist die Kraftverteilung zur Peripherie des Ankers. Es besteht eine unterschiedlich ausgeprägte Neigung z.B. der Schlundseitenwände zur Mitte hin zu kollabieren. Durch seine Form soll der Anker dem entgegenwirken. Der Anker, der vorzugsweise plattenförmig ausgestaltet ist, besitzt bevorzugt eine zum peripheren Bereich abnehmende Materialstärke. An Markierungen kann er entsprechend der individuellen anatomischen Situation gekürzt werden. Die Enden laufen bevorzugt schlank aus, um leichter in das Gewebe eingeführt werden zu können. Am Ende der Ankerflügel befinden sich die Anteile, die vom Operateur abgetrennt werden können, um individuelle anatomische Gegebenheiten zu berücksichtigen, durch geringere Materialstärke abgesetzt.

In bevorzugten Ausführungsformen stellt die Ankervorrichtung eine runde, elliptisch geformte, ovale Platte dar. Es können Perforationen unterschiedlicher Zahl und Größe vorhanden sein, um die Integration der Vorrichtung in das Gewebe zu verbessern. Ferner kann die Ankerplatte mit einem bildgebenden Material, beispielsweise Bariumsulfat, dotiert sein, um eine bildmorphologische bzw. radiologische Überprüfung zu erlauben, z.B. ob das Implantat in korrekter Position und intakt im Körper liegt. Die Ankerplatte kann mit Silber (Nanosilber) dotiert oder mit einem anderen Material beschichtet sein, das einer Kolonisation des Implantats und/oder einer Infektion des umliegenden Gewebes entgegenwirkt. Eine weitere Ausführungsform stellt ein Anker dar, der sich entsprechend dem Prinzip eines Regenschirms entfaltet. Dadurch kann das Implantat ohne offene Verbindung von der äußeren Wunde zum Beispiel am Kinn und Zungengrund eingepflanzt werden. Die Operationszeit ließe sich so verkürzen, was die Gefahr von Infektionen sowie die zu erwartenden Schmerzen minimiert. Die Flügel des Ankers sind dabei an ein elastisches Band angelegt und drängen in das Gewebe, wenn sie freigegeben werden.

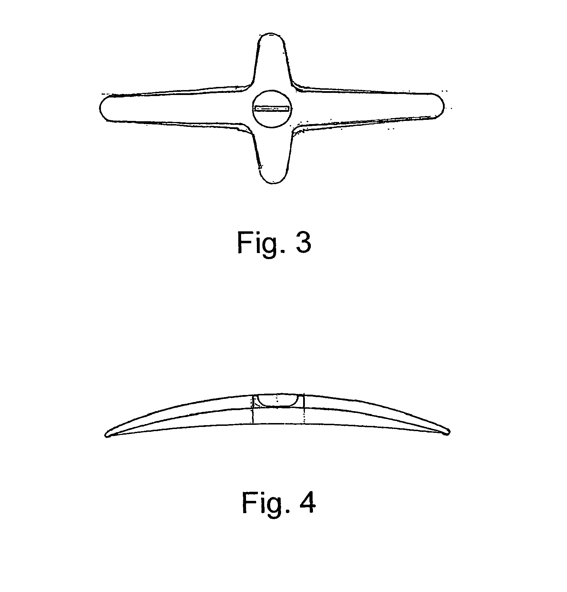

Bevorzugte Ausführungsformen der Ankervorrichtung sind in Figur 3 und 4 dargestellt. Die Enden der Ankervorrichtung könne spitz oder plump auslaufen. Scharfkantige Enden sind zu vermeiden, da sie eine Extrusion begünstigen. Spitze Enden erleichtern das Implantieren der Vorrichtung im Gewebe. Je weiter die erfindungsgemäße Ankervorrichtung seitlich dimensioniert ist, desto weicher und plumper müssen die Enden sein. Bei Implantation im beweglichen Teil der Zunge (Landmarke Linea terminalis) muss die Ankervorrichtung eine kleinere oder gleiche Breite wie die Zunge besitzen. Bei Implantation im unbeweglichen Teil der Zunge (Landmarke Glossotonsillarfurche) kann die erfindungsgemäße Ankervorrichtung breiter sein und sich an der Pharynxseitenwand oder der Rachenhinterwand abstützen. Ferner kann die Ankervorrichtung gerade oder gekrümmt nach vorne konkav oder konvex gekrümmt sein. Die craniocaudalen (senkrechten) Schenkel können gegensinnig gekrümmt sein. Die senkrechten Schenkel können unterschiedlich lang sein, sie können aber auch verkürzt, gegebenenfalls nur stummelförmig ausgebildet sein. Wenn die Ankervorrichtung nach vorne konvex geformt ist, stellt sie die angestrebte Form des Atemrohres dar und unterstützt diese. Die von der Seite zur Mitte hin wirkenden Kräfte werden allein durch die Steifigkeit des Materials aufgenommen. Auch ohne Zug nach vorne wird in diesen Grenzen der Atemweg offen gehalten. Je weiter die Vorrichtung zur Seite bzw. nach oben und unten reicht, desto mehr Gewebe wird am Kollaps gehindert. Die Krümmung der Vorrichtung kann der natürlichen Krümmung der Zunge nachempfunden sein, so dass keine Vorspannung der Vorrichtung erforderlich ist. Die Vorrichtung kann stärker gekrümmt sein und kann auch planar ausgeführt sein. Eine Ankervorrichtung mit konkaver Form wird erst durch den Zug des elastischen Bandes nach vorne in die gewünschte Form gebracht, so dass in diesem Fall das elastische Band ein unverzichtbarer Bestandteil der erfindungsgemäßen Vorrichtung ist. Die Vorspannung der Vorrichtung führt zu größeren Kräften an den Enden der Ankervorrichtung.

Gemäß einer weiteren Ausführungsform ist die Ankervorrichtung durch Ankerflunken gekennzeichnet, die so gestaltet sind, dass ihre nach vorne konkavbogige Form die Pharynxseitenwände aufspreizen, wenn der Zug in Richtung Kinnspitze zunimmt. Dadurch entsteht eine Kraft senkrecht zur Zugrichtung. Gemäß einer weiteren Ausführungsform sind die Ankerflunken so gestaltet, dass der Elastizitätsmodul an den Enden dem des umgebenden Gewebes entspricht und somit eine Extrusion vermieden wird. Dies kann beispielsweise dadurch erreicht werden, dass die Ankerflunken zu ihren freien Enden dünn auslaufen.

Gemäß einer weiteren Ausführungsform kann der Anker mit dem elastischen Band in einem Stück gefertigt sein.

Generell kann die Ankervorrichtung aus beliebigem Material sein, vorausgesetzt das Material ist biologisch kompatibel und für den dauerhaften Verbleib im Körper geeignet. Beispiele für Materialien sind Silikone, Polyurethan, Polytetrafluorethylen. Ein beispielhaftes Handelsprodukt ist das Material Nusil 4870 LSR Implantatsilikon. Da Silikone in verschiedenen Härtegraden angeboten werden (Shor 45 bis 70), kann das Implantat komplett aus einem Silikontyp oder in Teilen aus unterschiedlichem Silikon gefertigt sein. Das Band kann komplett aus einem elastischen Material, partiell oder komplett aus einem nichtelastischen Material gefertigt sein.

Generell kann der Anker Stabilität durch Materialeigenschaften, Materialstärken und -form erhalten. Eine Ausführungsform besteht darin, die Ankerflunken in einer der Zugrichtung entgegengesetzten Richtung zu krümmen. Die Annäherung an ein Kugelsegment verspricht die größte Steifigkeit bei geringstem Materialeinsatz. Weiterhin kann die gewünschte Materialsteifigkeit des Ankers und/oder des elastischen Elements durch gängige Verfahren der Statik, wie zum Beispiel Verstärkungsrippen, erzielt werden.

Die Größe der Ankervorrichtung wird im Wesentlichen durch die anatomischen Gegebenheiten an der jeweiligen Stelle bestimmt. Eine Ankervorrichtung zur Verwendung zur Positionierung eines menschlichen Zungenkörpers besitzt beispielsweise folgende Dimensionen, die der üblichen Variabilität des menschlichen oder tierischen Zungenkörpers entsprechen.

Liegt das elastische Element in Bandform vor, kann dieses mit der Ankervorrichtung verklebt sein. Die Ankervorrichtung kann auch einteilig mit dem bandförmigen elastischen Element gefertigt sein. Das bandförmige elastische Element kann am Zungengrund eine Verdickung aufweisen. Es kann in dieser Ausführungsform durch eine Öffnung der Ankervorrichtung hindurchgezogen werden und findet in einer Aussparung an der Ankervorrichtung Halt. Die Aussparung ist so gestaltet, dass das bandförmige elastische Element durchgleiten kann, die Verdickung am Ende jedoch nicht. So kann das elastische Element im Fall eines Defekts oder wenn eine andere Materialeigenschaft gewünscht ist gewechselt werden. Bandförmige elastische Elemente können unterschiedliche Shor-Härte und unterschiedliche Dimensionierung aufweisen. So kann je nach patientenspezifischer Anforderung die richtige Größe des elastischen Elements eingesetzt werden.

Das elastische Element in. Bandform kann unterschiedlich dimensioniert sein. Für die bei der Implantation in einem Zungenkörper zu erwartende Zugkraft von 1 N ist das fadenförmig ausgestaltete elastische Element 3,5 mm breit und 0,75 mm dick. Während des Eingriffs hat der Operateur die Möglichkeit, die Ankerflunken zu kürzen und so an die Anatomie des Zungengrundes anzupassen.

Das weitere Element des Systems, das Widerlager, kann in beliebiger technischer Realisierung vorliegen. Bevorzugt liegt es in Form einer Schraube vor. Diese sollte einen Tiefenanschlag besitzen, sodass der Operateur automatisch und zwangsweise die richtige Einschraubtiefe findet. Im freien Anteil sollte kein Gewinde sein, um das zu implantierende Widerlager an den scharfen Gewindegängen nicht zu verletzen. Die Schraube muss aus einem gewebeverträglichen und inerten Material sein, um gegen Abstoßungen weitestgehend sicher zu sein. Das bevorzugte Material ist Reintitan. Ferner sollte die Schraube selbstschneidend sein, um den operativen Aufwand klein zu halten (Gewindeschneiden). Sie muss kräftig genug sein, um nicht versehentlich abgedreht zu werden (≥ 2 mm Durchmesser) oder um keinen Ermüdungsbruch zu erleiden. Der Schraubenkopf sollte rund, oval oder rechteckig sein. Das elastische Element kann mit bevorzugt ebenfalls rechteckigen, runden oder konischen Aussparungen bei Längsstellung der Schraube einerseits leicht eingehängt werden, andererseits kann es bei querstehendem, also arretiertem Schraubenkopf nicht versehentlich abrutschen. Der Schraubenkopf ist für die Aufnahme eines handelsüblichen Schraubendrehers aus der Ostheosynthesechirurgie vorbereitet. Die Schraube kann auch hakenförmig ausgeführt sein. Die Art der als Widerlager verwendeten Schraube ist nicht kritisch und kann von einem Fachmann entsprechend der vorliegenden Offenbarung aufgrund seines Fachwissens leicht ausgewählt werden. Die Schraube sollte dauerstabil den Kräften, die auf sie übertragen werden, widerstehen. Ferner muss sie ausreichen groß dimensioniert sein, so dass sich das eingehängte elastische Element nicht lösen kann. Ferner muss sie aber ausreichend klein sein, um vom Patienten nicht als störend empfunden zu werden. Beispiele hierfür sind ein Schraubendurchmesser von ≥ 1 mm und ≤ 5 mm, einer Länge von ≥ 3 mm und ≤30 mm. Das Gewinde kann selbstschneidend oder nicht selbstschneidend sein. Das Loch, das die Schraube aufnimmt, wird vorgebohrt, das Gewinde geschnitten, die Schraube eingedreht. Die Schraube kann einen kreisrunden Kopf besitzen, was den Vorteil hat, dass sie preiswert und unkritisch zu produzieren ist und eine Fehllage durch falsches Eindrehen unwahrscheinlich ist. Ferner kann die Schraube aber einen in der Seitenansicht flach gestaltetem Kopf (Linsenkopf) besitzen. Der Kopf kann aber auch größer sein, was den Vorteil besitzt, dass hierdurch das elastische Element sicher vom Abgleiten gehindert wird. Eine Schraube mit in der Seitenansicht flach gestalteten Kopf (Linsenkopf) kann zusätzlich eine Beilagscheibe auf der erfindungsgemäßen Vorrichtung fixieren. Die Beilagscheibe kann alle Formen annehmen und aus beliebigen biokompatiblen Materialien bestehen. Bevorzugt besteht sie aus einer Folie. Die Folie muss steif genug sein, um ein Abgleiten des erfindungsgemäßen elastischen Elements zu verhindern und weich genug sein, um Irritationen des Patienten zu verhindern. Bevorzugt ist die Folie kreisrund ausgeprägt und hat einen Durchmesser, der die Breite eines beispielsweise bandförmig ausgestalteten elastischen Elements knapp überragt. Ein bevorzugtes Beispiel für eine Foliendimensionierung ist ein Durchmesser von 4 mm und eine Dicke von 0,5 mm.

Die Schraube kann einen in der Aufsicht symmetrischen, ovalen, rechteckigen oder elliptischen Kopf besitzen. Sie kann einen hakenförmigen Kopf besitzen. Der Schaft kann konusförmig oder zylinderförmig ausgestaltet sein.

Bevorzugt ist die Schraube am Kopf und an dem aus dem Knochen ragenden Teil mit Silikon überzogen (Beispiel Zahnimplantat).

Das Widerlager kann auch aus einem gestielten Silikonkopf, über den das Band mit seiner schlitzförmigen Öffnung eingehängt wird, bestehen.

Anstelle einer Schraube kann das Widerlager auch die Form einer einfachen Klemmung haben. Hierzu müsste das elastische Element eine strukturierte, zum Beispiel sägezahnartige, Oberfläche besitzen, um es in einer Richtung durch eine entsprechend geöffnete Klemmung zu ziehen und ein Zurückgleiten zu verhindern.

Durch die Schraube wird das eigentliche Widerlager am Knochen festgehalten. Das Widerlager besteht dabei aus einem Silikonblock, der zur Aufnahme des elastischen Elements eine Bohrung entsprechend exakt dem Banddurchmesser enthält. Mit einer Kappe, die den Block abdeckt und die einen zentralen Stift beinhaltet wird das Silikonband in der individuell festgelegten Position befestigt. Wenn eine Korrektur oder Reparatur erforderlich ist, reicht es aus, den Block freizulegen und die Kappe abzuziehen und das Band neu zu justieren. Nach Wiederaufsetzen der Kappe erfolgt der Wundverschluss. Die Kappe kann an den Kanten mit einer umlaufenden Lippe den Abschluss zum angeschraubten Block bilden. Die Kappe kann von kleinerer Fläche als der Block sein. Die Kappe kann in dem Block so eingelassen sein, dass die Oberfläche bündig abschließt. Der Stift, der an der Kappe angebracht ist und die Verbindung zwischen Block und Band herstellt, kann mit seinem freien Ende in einer Vertiefung des Blocks aufgenommen werden.

Das elastische Element kann in verschiedenen Materialstärken und Materialhärten verwendet werden. Auch Kombinationen von Materialien unterschiedlicher Härte (z.B. verschiedener Silikonhärten) können verwendet werden. Die Breite der Fläche und die Materialstärke können den Bedürfnissen entsprechend konfiguriert werden, um zum Beispiel das Implantat individuell Patienten mit unterschiedlichen Halsgrößen anzupassen.

Eine weitere mögliche Form des Widerlagers ist eine gekapselte Spule oder ein Exzenter über den das elastische Element gelegt wird. Über einen Schraubenkopf könnte dann über eine Stichinzision nachjustiert werden. Das Widerlager kann auch in Form einer elektromechanischen Verriegelung vorliegen, die nächtlich den Kraftschluss herstellt, tagsüber aber gelöst wird, um jeglichen Einfluss auf das zu positionierende Körperteil abzustellen.

In einer weiteren Ausführungsform kann das Widerlager auf den Knochen geschraubt werden oder in eine Lücke des Knochens integriert werden. Das Widerlager kann auch partiell in den Knochen integriert werden, d.h. ein Teil wird nicht in den Knochen integriert und ist mit dem elastischen Band verbunden, der andere Teil wächst in den Knochen ein. Ferner kann das Widerlager in einem Teil mit einer Schraube am Knochen befestigt werden. In einer weiteren Ausführungsform kann am Knochen ein Sockel mit einer oder mehreren Schrauben befestigt werden und das Widerlagers wird in den Sockel eingehängt und arretiert. Eine entsprechende Verschlusskappe kann zur Befestigung des Bandes in einer Ebene oder zur Befestigung des Bandes in zwei Ebenen dienen. Weiterhin kann das elastische Band durch einen oder mehrere Bolzen im Widerlager arretiert werden. Diese Bolzen können im Grundkörper oder in der Verschlusskappe befestigt sein. Die Verschlusskappe kann dabei vollständig vom Grundkörper getrennt sein oder mit ihm über eine Materialbrücke verbunden sein. In diesem Fall wird sie über diese Brücke, die als Scharnier dient, auf den Grundkörper gekippt.

In einer weiteren Ausführungsform besitzt die Verschlusskappe eine nach innen gerichtete Lippe, die das Eindringen von Flüssigkeit in das Widerlager verhindert und zur sicheren Befestigung der Kappe auf dem Grundkörper dient. Für die Justierung/Korrektur der Bandspannung wird kein Werkzeug aus dem Osteosyntheseinstrumentarium benötigt.

In einer weiteren Ausführungsform besitzt die Verschlusskappe einen nach außen gerichtete Lippe, die Eindringen von Flüssigkeit in das Widerlager verhindert und zur sicheren Befestigung der Kappe auf dem Grundkörper dient. Die Verschlusskappe kann mit Silikonkleber fixiert werden oder aufgeschraubt werden.

In einer weiteren Ausführungsform wird das elastische Band durch einen flüssigkeitsdichten Kanal des Widerlagers geführt. Am freien Ende wird ein Verschlussstück aufgeklemmt oder -geklebt. Dieses Verschlussstück kann passend über das Band gefädelt werden und an der vorgesehenen Position befestigt werden oder es wird über das Band geführt und befestigt. In dieser Ausführungsform besitzt das Band keine Kerben, Mulden oder Löcher.

Das Widerlager kann aus unterschiedlichen gewebeverträglichen Materialien bestehen. Bevorzugt werden bioinerte Materialien verwendet. Besonders bevorzugt ist Silikon unterschiedlicher Härtgrade, gegebenenfalls dotiert mit Röntgenkontrastmittel und/oder Nanosilber, Polyethylen, Polyamid, Polyester, Titan, anderen Metallen, Keramik, Verbundwerkstoffen, zum Beispiel auch in Beschichtung mit Gold.

In einer weiteren Ausführungsform kann das Widerlager auch mit einer selbstdichtenden Titanschraube und philiformen Löchern des elastischen Bandes realisiert werden. Hierbei fasst die das elastische Band verriegelnde Schraube ein Gewinde, das im Schraubenkopf der Schraube untergebracht ist, die den Widerlagerblock am Knochen fixiert.

Die Größe des Widerlagers entspricht dem zu positionierenden Körperteil, beispielsweise beträgt die Größe des Widerlagers für eine Vorrichtung zur Positionierung eines Zungenkörpers, z.B. eine Schraube 8 mm in der Länge und 2 mm im Durchmesser. Ein Schraubenkopf hat beispielsweise einen kleinsten Durchmesser von 4 mm und einen größten Durchmesser von 7 mm.

Die erfindungsgemäße Vorrichtung hat bei Implantation in einem Zungenkörper den Vorteil, dass der Anker den seitlichen Pharynx aufspannt und auch nach cranial und caudal einen Zug nach vorne ausübt. Damit wird der Kollaps in drei Dimensionen verhindert. Durch Spannen des elastischen Elements, d.h. des Ankerbandes, kann der Operateur die der Schwerkraft entgegengesetzte Kraft verändern. Mit einem mitgelieferten Kraftmesser (Dehnungsmesser oder Federwaage) kann eine Grundeinstellung vorgenommen werden. Durch Inspektion, z.B. mit optischen Hilfsmitteln (flexible Faseroptik, starre Winkeloptik), kann die Bewegung des Zungenkörpers weg von der Rachenhinterwand beobachtet werden. Ist der Patient in Narkose relaxiert, kann die Maximalvariante der schlafbezogenen Muskelerschlaffung, die das pathogenetische Prinzip der Schlafapnoe ist, simuliert werden. So kann die Spannung des elastischen Moduls so eingestellt werden, dass die Zunge von der Rachenhinterwand abhebt. Bei gegebenenfalls erforderlichen Korrekturen besteht die Möglichkeit, das elastische Element an einer anderen Position am Widerlager an der Schraube einzuhängen und damit die Kraft zu erhöhen oder zu verringern.

Die erfindungsgemäße Vorrichtung kann auch als Ausführungsform hergestellt werden, bei der magnetismus zur justierbaren Positionierung des Körperteils genutzt wird. Hierbei liegt die Abstoßung zweier oder mehrerer Dauermagneten zwischen den zu positionierenden Körperteilen, im Falle der Zunge zwischen der Rachenhinterwand und dem Zungenkörper, oder die Anziehung zweier oder mehrerer Dauermagneten zwischen den gegenüberliegenden Körperteilen, im Falle der Zunge der Unterkieferspitze und dem Zungengrund, vor. Bevorzugt liegt hier das elastische Element in Form von magnetischen Scheiben oder Quadern vor. Das Widerlager ist ein kompakter Magnet im Bereich der Wirbelsäule. Die magnetischen Scheiben oder Quader können mit entgegengesetzter Polarität unmittelbar unter die Schleimhaut an korrespondierenden Stellen der zu positionierenden Körperteile eingebracht werden. Die Abstoßung der magnetischen Körper verhindert eine Annäherung der Körperteile in den von der magnetischen Kraft vorgegebenen Grenzen. Im Falle der Positionierung eines Zungenkörpers übersteigt die Muskelkraft der Zunge die Abstoßungskraft der Magneten, sodass Schlucken und Sprechen nicht behindert werden. Die magnetischen Körper können aus jedem beliebigen magnetisierbaren Material vorliegen. Bevorzugt sind sie in ein gewebeverträgliches Material, zum Beispiel Silikon, eingebettet und können mit Wirkstof fen, die die Integration in das Gewebe fördern und/oder bakteriostatisch/bakterizid sind, beschichtet sein. Ferner können sie dotiert sein, um in bildgebenden Verfahren dargestellt werden zu können.

Beispiele für magnetische Körper sind Legierungen aus Aluminium, Nickel und Kobalt (AlNiCo), Ferritmagnete (d.h. gesintertes und danach magnetisiertes Gemisch aus Barium- oder Strontiumoxid mit Eisenoxid), Legierungen aus dem Seltenerdmetall Samarium und aus Kobalt (SmCo) oder Legierungen unter Verwendung von Neodym, beispielsweise die Legierung NdFeB (bestehend aus Neodym, Eisen und Bor), die zur Verbesserung der Eigenschaften zu Pulver gemahlen, gepresst, gesintert und mit einer Oberflächenbeschichtung versehen werden kann, um Korrosionen vorzubeugen. Es ist günstig, wenn die Koerzitivfeldstärke (was für die Entmagnetisierbarkeit oder die Tendenz eines Permanentmagneten durch äußere Magnetfelder die magnetische Kraft zu verlieren) relativ hoch ist. So besitzt beispielsweise ein Ferritmagnet eine drei- bis fünffach höhere Koerzitivfeldstärke als eine Legierung aus Aluminium, Nickel und Kobalt und Legierungen unter Verwendung von Samarium besitzen eine ca. 14-mal so hohe Koerzitivfeldstärke wie eine Legierung aus Aluminium, Nickel und Kobalt. Magnete unter Verwendung von Neodym besitzen eine ca. 17-mal so hohe Feldstärke wie Legierungen aus Aluminium, Nickel und Kobalt.

Bevorzugt werden moderne Permanentmagnete verwendet. Diese sind im Gegensatz zu Ferromagneten annähernd unbegrenzt haltbar, wenn sie in geeigneten Temperaturgrenzen aufbewahrt werden. Dies ist bei der menschlichen Körpertemperatur in jedem Fall gegeben. Es lässt sich die Abstoßungs- und Anziehungskraft ausnützen. Bei Verwendung der Abstoßungskräfte durch das vollständig implantierte erfindungsgemäße System werden zwei Permanentmagnete so implantiert, dass die Magnetkraft sich auf die Körpergewebe überträgt, deren Annäherung zu Krankheitserscheinungen führen würde. Implantation in den Zungengrund und in die Rachenhinterwand führt zu einer Abstoßung mit Bewegung des Zungenkörpers nach vorne. Je näher umgekehrt die Zunge der Rachenhinterwand kommt, desto größer wird die abstoßende Kraft. Die Magneten werden günstigerweise so gewählt, dass in Ruhelage der Zunge die abstoßende Kraft vernachlässigbar klein und durch den Patienten nicht spürbar ist.

Die Magnete müssen ferner in einer biokompatiblen Form vorliegen, um den in der Fachwelt bekannten Problemen bei Inkorporation von körperfremden Materialien wirksam zu begegnen. Die Magnete müssen ferner gas- und flüssigkeitsdicht eingebettet sein und können in bioinerte oder bioaktive Materialien eingebettet oder mit diesen überzogen sein. Beispielhafte Materialien sind Silikone unterschiedlicher Shorhärte mit Eignung zur Dauerimplantation, Polyethylen, Polyurethan, Polyester, PTFE, Keramik. Das Material muss vorzugsweise weich und elastisch sein, um einer Extrusion vorzubeugen. Ferner soll das Material die oben geforderten Materialeigenschaften bei möglichst kleiner Materialstärke aufweisen. Bevorzugt ist der Magnet scheidenförmig mit einer Dicke von 2 mm und einem Durchmesser von 6 mm eingeschweißt in Silikon Nusil 4845. Die Materialstärke über dem Magneten beträgt 0,5 mm, die maximale Materialstärke 2 mm, zu den Enden auf 0,2 mm auslaufend. Die Implantatlänge beträgt bevorzugt 15 mm und besitzt eine Breite von 7 mm. Der Magnetträger (Silikon) kann beliebige Formen annehmen. Er kann kreisrund, oval, elliptisch, kreuzförmig, sternförmig, rechteckig, quadratisch oder unregelmäßig geformt sein. Bevorzugt bedingt seine Geometrie eine optimale Verankerung im Gewebe. Neben scheibenförmigen Magneten können Magnete beliebiger Form Verwendung finden. Typischerweise sind Permanentmagnete quader-, kugel- oder stabförmig. Geeignete Magnetgrößen sind insbesondere neben Scheiben flache Quader, z.B. in einer Größe von 20x20x3 mm oder 5x5x1,5 mm.

Statt eines Magneten können auch mehrere Magnete in ein Trägermaterial eingesetzt werden. Sie können in diesem Trägermaterial so angeordnet sein, dass sie dem zweiten Trägermaterial an korrespondierenden Stellen entsprechen. Es können ein, zwei oder auch ein Array von Magneten vom Träger aufgenommen werden. Dabei muss die Abstoßungskraft ausreichend hoch sein, was eine Mindestgröße des Magneten bedingt. Ferner darf die zu implantierende Vorrichtung eine verträgliche Maximalgröße nicht überschreiten.

Es können mehr als zwei Träger mit Magneten implantiert werden. Statt zwei großen können mehrere kleine Träger implantiert werden. Durch eine geeignete Auswahl der Größe (Volumen) und der Remanenz der Magneten kann auf die unterschiedlichen Patientenbelange abgestellt werden. Beispielsweise entwickelt bei einer Distanz von 16 mm ein Neodymeisenbor-(NdFeB) Permanentmagnet über eine Dimensionierung von 20x20x3 mm eine dauerhafte Abstoßungskraft von 1 N. Bei Implantation der erfindungsgemäßen Vorrichtung im Rachenraum können in die Schlundseitenwand und in den Zungenrand weitere Magnetsysteme im Trägersystem implantiert werden. Als Bezugspunkt für die Magnetwirkung dient beim vollständig implantierbaren System ein Träger unter einer Weichgewebedecke der Rachenhinterwand. Dieser Bezugspunkt kann auch außerhalb des Körpers liegen. Dies ist der Fall bei einem teilweise implantierten System. In diesem Fall trägt der Patient einen im Zungengewebe implantierten Magneten. Die auf diesen wirkende abstoßende Kraft eines weiteren Magneten (Permanent- oder Elektromagnet), der sich außerhalb des Körpers dorsal der Zunge befindet, hält die Zunge in einem Abstand, der das Auftreten von Apnoen verhindert.

Es kann ein Magnet oder eine beliebige Anzahl von Magneten Anwendung finden: Das entscheidende Kriterium ist, dass die Summe der wirkenden Kräfte auf den implantierten Magneten ausreicht, um durch Annäherung der Körperteile, beispielsweise der Zunge und der Rachenhinterwand, hervorgerufene Symptome, beispielsweise Apnoen, Hypopnoen und Schnarchen, wirkungsvoll zu verhindern. Wesentlich ist, dass durch Form, Volumen und Anzahl der Magnete ein im jeweiligen Zielgebiet möglichst homogenes Magnetfeld erzeugt wird.

Da die Magnetkraft im Falle eines Zungenimplantats ausschließlich im Schlaf wirken muss, hätte diese Ausführungsform den Vorteil, dass sich die Zunge im Wachzustand frei von jeglichen Fremdeinflüssen bewegen kann. Der Magnet au-βerhalb des Körpers könnte in ein Hals-/Kopfband integriert sein oder sich im Kopfkissen des Patienten befinden. Die Fixierung an den Kopf des Patienten hat den Vorteil, dass die Kraft stets in derselben Richtung und Größe wirkt.

Das elektromagnetische Feld kann auch apnoegetriggert geregelt werden. Beispielsweise wird bei über mehreren Atemzyklen gemittelten nachlassendem frühinspiratorischem Flow das Feld so lange hochgeregelt, bis die Atemtiefe wieder eine individuell definierten Wert erreicht hat. Die Regelcharakteristik kann linear oder progressiv sein. Die Aufnahme der Soll- und Istwerte kann über Thermo- oder Akustiksensoren oder über einen Pneumotachographen unterstützt durch eine rechnergestützte Echtzeitsignalverarbeitung und Abgabe der Steuersignale an eine Verstärker erfolgen.

Beispielsweise wird eine Silikonträger mit zwei scheibenförmigen Magneten (8 mm Durchmesser, 3 mm Dicke) in den Zungengrund des Patienten implantiert. Nachts wird ein elastisches und gepolstertes Band, das für die Aufnahme von drei Permanentmagneten der Dimensionierung 20x20x13 mm geeignet ist, angelegt. Die Form des Bandes lässt ausschließlich die gewünschte Position am Kopf des Patienten zu. Eine Strangulation wird sicher ausgeschlossen. Die abstoßende Kraft auf den Zungengrund beträgt bei einem Halsumfang von 42 cm ca. 1 N.

Statt eines Bandes zur Aufnahme des externen Magneten kann auch ein vorgeformtes Kopfkissen so mit dem Magneten bestückt werden, dass ihre Kraft auf möglichst kurzer Strecke auf die implantierten (= internen) Magnete wirkt. Kopfkissen und Nackenrollen, die den Kopf in Grenzen fixieren, sind auf dem Markt erhältlich, z.B: Pilo^{®}, Nackenstützkissen Cervical von Dormabell, Kubivent Neck. Statt eines Bandes kann auch eine nach vorne offene Klammer verwendet werden, die die Magnete auf einer Schiene in verschiebbaren Positionen trägt. Die Klammer kann bezüglich Form und Haltefunktion einem Kopfhörer entsprechen. Die gepolsterten Auflagen sind verstellbar. Sie können beiderseits des Jochbogens aufliegen. Der magnettragende Haltebügel liegt an der Nacken-, Kopf- und Halshaut an.

Durch geeignete Auswahl der Größe (Volumen) und Remanenz des Magneten kann auf unterschiedliche Patientenbelange abgestellt werden, ohne dass ein Eingriff erforderlich wäre. Die ideale Magneteigenschaft könnte im Schlaflabor mit einem frei einstellbaren Elektromagneten ermittelt werden. Der Permanentmagnet würde entsprechend den so erhaltenen Ergebnissen ausgewählt und dem Patienten verordnet werden. Im Kopfteil des Patientenbetts/dorsal der Zunge im Falle einer gewünschten Abstoßung, anterior der Zunge im Falle einer gewünschten Anziehung, befindet sich ein Elektromagnet, der ein über Zeit und Ort gleichmäßiges elektromagnetisches Feld erzeugt.

An der gleichen Stelle befindet sich ein Elektromagnet, der ein über Zeit und Ort gesteuertes elektromagnetisches Feld erzeugt.

Die Steuerung/Regelung wird von der normalen Atemmechanik getriggert.

Das elektromagnetische Feld wird nur bei Inspiration eingeschaltet.

Die Steuerung/Regelung kann auch von der pathologischen Atemmechanik getriggert werden. Das elektromagnetische Feld wird nur bei Apnoen/Hypopnoen/ Schnarchen eingeschaltet.

Die Magnete können ferner im Kragen eines speziell angefertigten Schlafanzugoberteils eingearbeitet sein. Der Kragen zeichnet sich gegenüber handelsüblichen Schlafanzugoberteilen dadurch aus, dass er steif genug ist, um nicht umzuknicken. Er ist hautverträglich und auf der Innenseite weich, um bei einem geforderten engen Kontakt nicht zu Unverträglichkeitsreaktionen zu führen. Er besitzt eine Verbindung zur Schulter-, Rücken- und Brustpartie, die steif genug ist, eine kalkulierbare Position des magnettragenden Kragenanteils zu gewährleisten. Er besitzt eine Verbindung zur Schulter-, Rücken- und Brustpartie, die hoch genug ist, um eine Positionierung des magnettragenden Kragenanteils möglichst auf Höhe des Zungengrundes zu gewährleisten. Der Kragen ist mit handelsüblichen Verfahren abnehmbar (beispielsweise Klettverschluss, Reißverschluss, Knöpfe, eingearbeitete Tasche). Das Schlafanzugoberteil ist waschbar.

Soll die Anziehungskraft zwischen den beiden Magnetelementen ausgenutzt werden, liegt der magnettragende Fixpunkt an einer knöchernen Struktur des Mittelgesichts oder Unterkiefers. Die Anziehungskraft eines z.B. am Unterkiefer fixierten Magneten wirkt auf einen im Zungengewebe und/oder im Gewebe der Schlundseitenwände implantierten Magneten bzw. mittelbar auf den Magnetträger. Dabei ist es auch möglich, den Magnetträger so zu gestalten, dass die Kraft von einem nicht der Zungeoberfläche nahen Magneten auf ein Implantat übertragen wird, welches die Kraft am Zungengrund vermittelt. Dies hätte den Vorteil, dass die Magneten näher benachbart wären und somit die zwischen ihnen wirkenden Kräfte größer wären. Ein Nachteil dieser Ausführungsform ist, dass mit zunehmender Enge des Atemrohres die Kräfte kleiner werden. Die Magnete könnten zur Behandlung des Schnarchens auch am Weichgaumen implantiert werden.

Die Magnete sollen klein sein, um als Fremdkörper nicht oder möglichst wenig wahrgenommen werden zu können. Sie sollen in ihrer Polung exakt definiert und unveränderlich im Gewebe fixiert sein. Ferner sollen sie ausreichend viel Gewebevolumen beeinflussen, um das Gewebe wirksam auf Abstand zu halten. Sie sollen ausreichend knapp unter den sich annähernden Oberflächen positioniert werden, um die größtmögliche Kraft aufeinander entwickeln zu können. Ferner sollen sie keine unerwünschten Nebenwirkungen zeigen und sind bei Trägern implantierter telemetrischer Apparate, wie z.B. Herzschrittmacher, Shuntventile, ggf. kontraindiziert. Patienten, die absehbar einer Kernspintomographie bedürfen, sind entsprechend zu informieren und von der Implantation auszunehmen.

Mit der implantierbaren Vorrichtung unter Verwendung der Magnetkraft lassen sich beliebige Körperteile positionieren. Beispielsweise kann bei Lidparese die Anziehung zweier Magnetsysteme, die in den Lidkanten oder lidkantennah implantiert werden, ausgenützt werden. Das Widerlager ist für diese Anwendung das Unterlid. Statt der Nahtfixation des Oberlides und der Augenbraue bei Lähmung des M. levator palpebrae ist ein Magnetsystem ebenfalls verwendbar. Hier ist das Widerlager am Stirnbein.

Bei der durch die Fazialisparese verursachten Ptose des Mundwinkels dient ein Magnet am Oberkieferknochen als Fixpunkt/Widerlager, der zweite/die anderen in ein Trägersystem integrierten und entweder direkt am Mundwinkel oder über eine Schlinge mit dem Mundwinkel in Verbindung stehend bewegen sich relativ zum Fixpunkt.

Im Bereich der Nasenklappe werden die abstoßenden Kräfte zweier oder mehrerer Magnete ausgenützt. Diese werden im Bereich des Limen nasi so implantiert, dass die Abstoßung zu einer ausreichenden Erweiterung führt.

In jedem Fall muss das Trägersystem so gestaltet sein, dass die Magnete auch im langen Zeitverlauf keine Bewegung um ihre eigene Achse ausführen können. Dies ist insbesondere immer dann von entscheidender Bedeutung, wenn die Abstoßung ausgenützt wird.

Von besonderer Bedeutung ist die Positionierung von Körpergewebe bei Patienten mit Inkontinenzproblemen. Sowohl bei der Stuhl- als auch bei der Harninkontinenz kann durch die erfindungsgemäße Vorrichtung insbesondere unter Verwendung der Magnetkraft ein Verschlussmechanismus erzielt werden, der durch einen bestimmten Öffnungsdruck überwunden werden kann.

Sämtliche Teile der erfindungsgemäßen Vorrichtung können in einem Standardverfahren für Präzisionsspritzgussteile in an sich bekannter Weise hergestellt werden. Entsprechende Verfahren sind einem Fachmann gut bekannt. Je nach Modellform werden ein oder mehrere Nester gefräst. Diese werden dann mit der Silikonmasse befüllt ("Kavitätenspritzgussform"-Verfahren).

Ferner kann die erfindungsgemäße Vorrichtung auch nach der Funkenerodierungsmethode hergestellt werden. Dieses einem Fachmann bekannte Verfahren besitzt im Gegensatz zu spanenden Verfahren den Vorteil, wesentlich höhere Oberflächengüten zu erzeugen. Die Erzeugung einer besonders glatten Oberfläche ist für implantierbare Teile von großer Bedeutung. Je glatter die Oberfläche ist, desto kleiner ist die Fläche, die Entzündungsreaktionen und enzymatischen Angriffen ausgesetzt ist. Ebenso sinkt damit der Widerstand der Relativbewegungen und damit der Verschleiß. Dies verringert die Gefahr einer bakteriellen Besiedelung.

Die folgenden Figuren erläutern die erfindungsgemäße Vorrichtung näher:

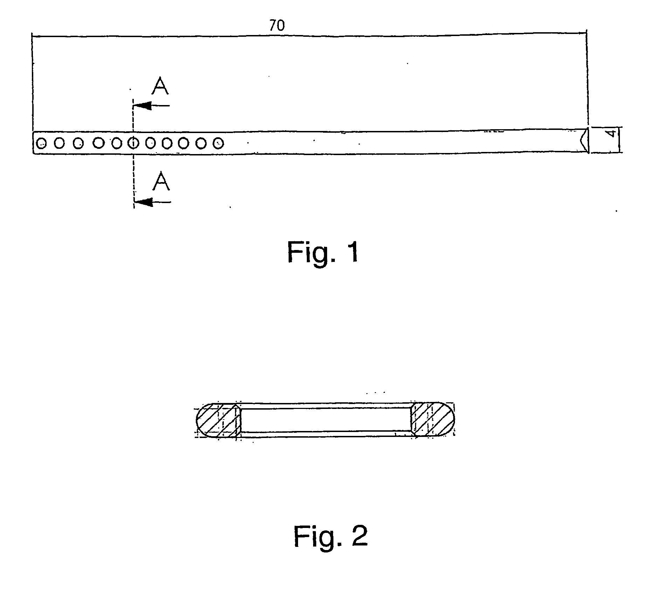

- **Figur 1:**: Elastisches Element in Form eines Bandes. Die Angaben sind in [mm]. Rechts ist die Verdickung mit 4 mm Breite und 2 mm Höhe eingezeichnet, die das Ausrauschen aus der Ankerplatte verhindert. Schnitt AA zeigt ein Loch im Band, das den Arretierungsstift bzw. die Schraube des Widerlagers aufnimmt.
- **Figur 2:**: Schnitt AA aus Figur 1.
- **Figur 3:**: Ankerplatte in der Draufsicht.
- **Figur 4:**: Ankerplatte im Querschnitt.

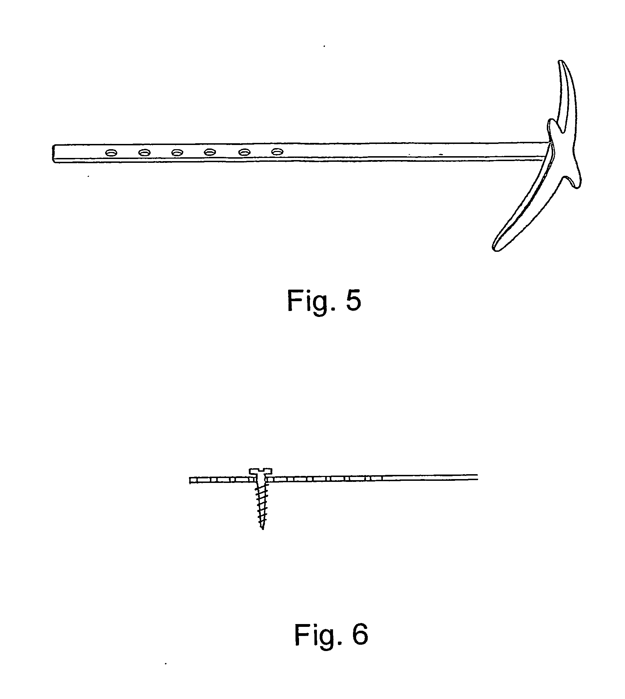

- **Figur 5:**: Anker mit elastischem Band verbunden.
- **Figur 6:**: Elastisches Band mit Schraubverbindung zum Knochen als Widerlager.

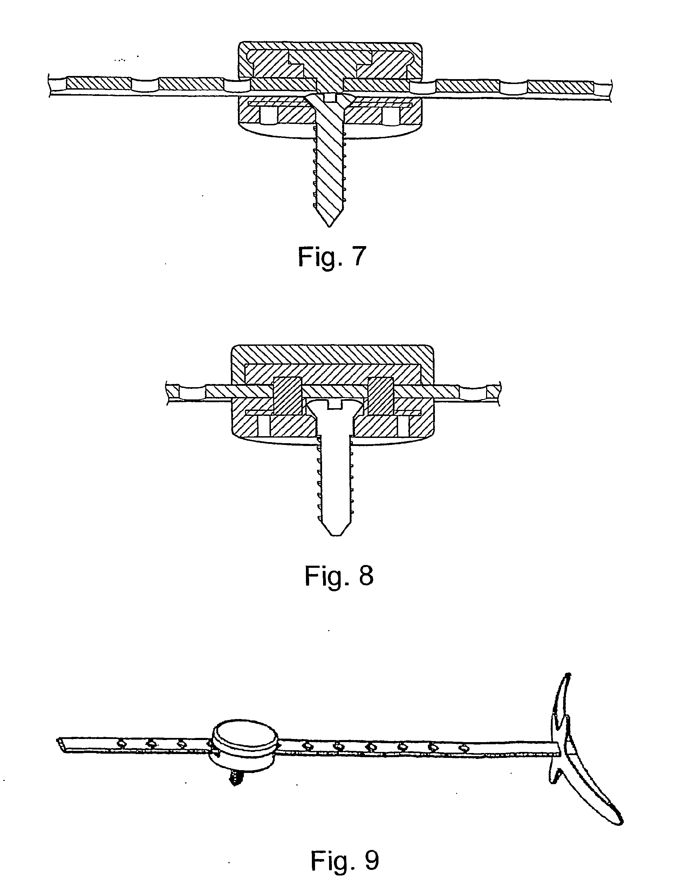

- **Figur 7:**: Widerlager im 3D-Querschnitt.

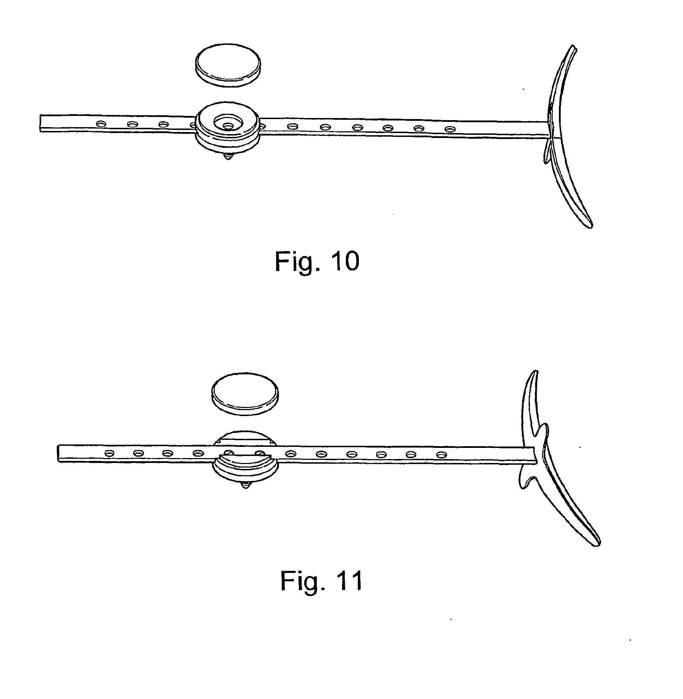

- **Figur 8:**: Querschnitt des Widerlagers gemäß Fig. 11 in der 3D-Aufsicht.
- **Figur 9:**: Implantierbare Vorrichtung mit komplett geschlossenem Widerlager und elastischem Band in der Schrägaufsicht.
- **Figur 10:**: Entspricht Fig. 9 mit komplett geöffnetem Widerlager in der 3D-Schrägaufsicht.
- **Figur 11:**: Ausführungsform des Widerlager, bei dem aus dem geöffneten Widerlager das Band gehoben werden kann. Die Verschlusskappe dient der Fixierung des Bandes im Führungskanal in zwei Ebenen. Der Verriegelungsbolzen ist nicht in der Kappe, sondern im Grundkörper integriert. Die Abbildung zeigt das komplett geöffnete Widerlager in der 3D-Schrägaufsicht.

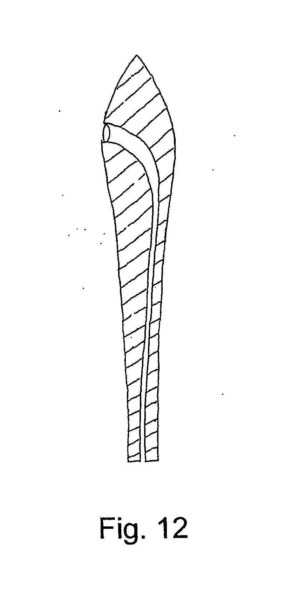

- **Figur 12:**: Trokar mit Führungskanal; Austritt für den Führungskanal am verdickten Ende, Einschuböffnung am der Spitze gegenüberliegenden Ende.

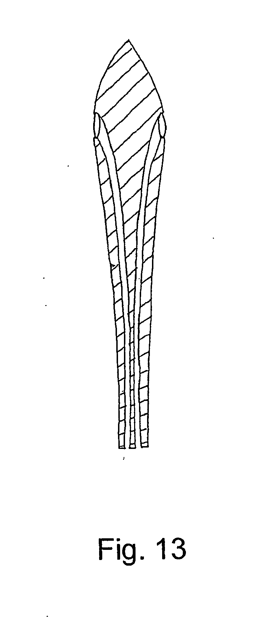

- **Figur 13:**: Trokar mit zwei Führungskanälen.

Die erfindungsgemäße Vorrichtung wird zur Positionierung eines menschlichen oder tierischen Körperteils dadurch verwendet, dass die Vorrichtung in die entsprechenden Körperteile implantiert wird.

Die erfindungsgemäße Vorrichtung eignet sich zur Positionierung eines Zungenkörpers.

Besonders vorteilhaft ist die erfindungsgemäße Vorrichtung zur Positionierung der Zunge in physiologischen Grenzen. Dies ist dadurch gegeben, dass der Anker am Zungengrund über ein elastisches Band und ein Widerlager am Unterkiefer eine Kraft in sagittaler Richtung zur Kinnspitze hin ausübt. Die erfindungsgemäße Vorrichtung gewährleistet eine zuverlässige, einfache operative Behandlung des Schlafapnoe-Syndroms. Durch die kalkulierbaren Systemeigenschaften werden uneinheitliche Operationserfolge vermieden.

Das nachstehend beschriebene Operationsverfahren bezieht sich auf die Implantation der Vorrichtung in einen menschlichen Zungenkörper/Mundboden. Dabei wird der Patient mit rekliniertem Kopf gelagert. Nach Desinfektion der Halshaut, der Mundschleimhaut und der Zähne wird ein bevorzugt Epinephrin-haltiges Lokalanästethikum submental und im geplanten Stichkanal hin zum Zungengrund gegeben. Dann wird ein ca. 3 cm langer, frontaler Hautschnitt 1 bis 2 cm von der Kinnspitze entfernt vorgenommen. Es wird auf den Unterkieferknochen präpariert, das Periost wird abgelöst. Ferner wird das Widerlager am Unterkiefer so befestigt, dass es vom Patienten später nicht als störend empfunden wird.

Mit dem Zielbogen wird die Ausstichstelle am Zungengrund eingestellt. Der Trokar wird durch den Zielbogen in den Zungenkörper und am Zungengrund in Höhe der Linea terminalis (Papillae vallatae) ausgestochen. Von dieser Stelle ausgehend wird die Schleimhaut des Zungengrundes unterminiert, um dem Implantat Raum zu geben. Hierzu wird eine gebogene Schere oder ein für diesen Zweck entwickeltes Winkelmesser verwendet. Von außen kommend wird bevorzugt ein geflochtener oder kräftiger Faden bzw. Draht mit den Maßen 2 x 0 oder 0 durch den Trokar eingefädelt, am Zungengrund aufgefangen und am Implantat befestigt (Knoten, Steckverbindung). Von außen wird der Faden unter Zug gesetzt, gleichzeitig wird der Trokar entfernt. Auf diese Weise wird der Ankerfaden nach außen geführt. Die Ankerflügel werden in den Schleimhauttunnel eingeführt, die Schleimhaut wird bevorzugt mit 3 x 0 oder 4 x 0 Vicryl-Einzelkopfnähten verschlossen. Das lmplantat wird stark gespannt, um seine Festigkeit zu prüfen und den geradlinigen Verlauf des Fadens sicherzustellen: Nun wird das Implantat so an der Schraube eingehängt, dass ein Zug nach vorne entsteht, der die Zunge des in Rückenlage befindlichen und relaxierten Patienten von der Rachenhinterwand abhebt oder mindestens eine nach vorne gerichtete Kraft von 1 N am Kraftmesser abgelesen werden kann. Dieser wird in eine der freien Vertiefungen des Implantates eingehängt. Das Implantat wird um einen Betrag gekürzt, der ein Nachlassen der Spannung um das eingestellte Maß noch ermöglicht. Es erfolgt bevorzugt ein zweischichtiger Wundverschluss mit 4 x 0 Vicryl-Subcutan-Einzelkopfnähten, 4 x 0 oder 5 x 0 Hautnaht. Bei männlichen Patienten ist diese vorzugsweise als fortlaufende Intracutannaht ausgebildet, um kein Hindernis bei der Rasur zu erzeugen. Die Wunde wird verbunden. Intra- und postoperativ, jedoch mindestens perioperativ erhält der Patient ein Antibiotikum (zum Beispiel Cephalosporin). Es erfolgt eine postoperative Überwachung, mindestens mit Sauerstoffmonitoring, sowie eine laborchemische Kontrolle der Entzündungsparameter.

Gemäß einer weiteren Ausführungsform der Operationstechnik wird das elastische Element in Seldingertechnik eingebracht. Dabei wird ein Trokar von submental bis in den submukösen Raum vorgeschoben. Ein Draht wird durch den Trokar vorgeschoben und durch die Form des Trokarinneren beim Verlassen des Trokars zur Seite abgelenkt. Der Draht dringt durch die Muskulatur bis zum Rand der Zunge. Der Trokar wird entfernt. Über den Draht wird ein elastisches Modul so weit vorgeschoben, bis die Zielregion erreicht ist. Der Draht wird entfernt. Über den neuerlich eingeführten Trokar, der um 90° und/oder 180° in seiner Längsrichtung gedreht wurde, wird die oben beschriebene Prozedur wiederholt bis ausreichend elastische Elemente in der Zunge platziert sind, um den erforderlichen Zug an ausreichend Zungengewebe Richtung Kinnspitze vermitteln zu können. Die auf diesem Weg in die Zunge eingebrachten Körper können in ihrer Elastizität nachträglich beeinflusst werden, zum Beispiel durch Korrektur einer Vorspannung, Einbringen eines Kernes oder durch thermische oder chemische Verfahren.

Statt eines Trokars mit nur einer Führungsschiene kann auch ein Trokar verwendet werden, der alle erforderlichen Führungsschienen beinhaltet. Ein entsprechend größerer Außendurchmesser müsste in Kauf genommen werden, dafür fiele aber das Trauma durch wiederholtes Einführen weg.

Gemäß einer weiteren Ausführungsform der Operationstechnik ist für jedes einzubringende elastische Element ein eigener Kanal vorzusehen. Der Trokar kann in situ verbleiben. Die Spitze besteht idealerweise aus resorbierbarem Material. Es können auch die Wandungen des Trokars aus resorbierbarem Material bestehen.

Sofern ein Magnetträgersystem zu implantieren ist, werden die Magnetträger bevorzugt submukös, subfaszial oder intramuskulär implantiert. Um den minimal invasiven Charakter der Methode zu bewahren, hat sich folgendes Verfahren bewährt: Es wird ein 1,5 cm langer kraniokaudaler Schleimhautschnitt lateral im Pharynx vorgenommen, mit einem dafür bereitgestellten gewinkelten Messer zur gewünschten Länge unterminiert. Ein kräftiger Faden wird durch den Gewebetunnel geführt. Der angehängte Träger wird soweit durchgezogen, bis er komplett in der Wunde liegt. Der Faden wird gekappt, die Schleimhautwunde wird verschlossen. Am Zungengrund wird analog vorgegangen.

Mit der beschriebenen Implantation der erfindungsgemäßen Vorrichtung können insbesondere obstruktive schlafbezogene Atmungsstörungen wirksam behandelt werden. Weitere Indikationen für eine Implantation der erfindungsgemäßen Vorrichtung sind das "upper airway resistance syndrome", obstruktives Schnarchen, "heavy snorer syndrome", Glossoptose, Makroglossie bei Trisomie 21, krankhafte Vergrößerungen der Zunge, zum Beispiel der Akromegalie als Folge einer Hypophysenstörung.

Die nachstehenden Beispiele erläutern den Gegenstand der Erfindung näher.

### Therapiebeispiele:

Die nachstehend mit der erfindungsgemäßen Vorrichtung behandelten Patienten hatten entweder einen nCPAP-Versuch erfolglos hinter sich oder lehnten die nCPAP-Behandlung ab. In allen Fällen war ein Schlafapnoe-Syndrom polysomnographisch gesichert worden. Es lagen keine anatomischen Anomalien, schwere Fehlbisssituationen oder raumfordernde Prozesse der oberen Atemwege vor. Die Patienten wurden in mindestens zwei Aufklärungsgesprächen schriftlich über den Heilversuch in Kenntnis gesetzt. Das schriftliche Einverständnis der Patienten wurde eingeholt.

### Beispiel 1

Der Patient litt seit vielen Jahren an ausgeprägter Tagesmüdigkeit, zeigte eine Einschlafneigung am Steuer und bei der Arbeit und klagte über eine Einschränkung der Lebensqualität. Mehrfache konservative Therapieversuche (nCPAP) erwiesen sich als vergeblich.

Die erfindungsgemäße Vorrichtung wurde entsprechend der vorstehenden Beschreibung implantiert. Das erste Implantat bestand aus einem Silikon-Trachealstent (Dumon) und einer Silikontränenwegsschienung (Guibor Tränenwegsplatzhalter). Die Befestigung am Unterkiefer erfolgte mittels Mitek-Anker und die Knotenverbindung wurde mit den Silikonfäden hergestellt, die doppelt verwendet wurden. Der Heilungsverlauf erwies sich als unkompliziert.

Auf eigenen Wunsch verließ der Patient das Krankenhaus vorzeitig und begann unmittelbar darauf wieder zu arbeiten. Er fühlte sich deutlich leistungsfähiger und wacher, zeigte keine Einschlafneigung mehr tagsüber und berichtete über eine verbesserte Lebensqualität.

### Beispiel 2

Der Patient wies eine langjährige Krankengeschichte eines Schlafapnoe-Syndroms mit Morgenschläfrigkeit, Tagesmüdigkeit, Einschlafneigung und Leistungsschwäche auf. Er hatte mehrere erfolglose nCPAP-Versuche und Rhinochirurgie hinter sich.

Die erfindungsgemäße Vorrichtung wurde gemäß der vorstehenden Beschreibung implantiert. Im Gegensatz zum vorstehenden Beispiel bestand das elastische Element aus einem Silikonband aus der Netzhautchirurgie. Die Verbindung zwischen Ankerplatte (aus zurechtgeschnittenem Trachealstent) und dem Silikonband erfolgte mittels Goretex-Faden. Am Unterkiefer wurde die Verbindung mit einer Titanschraube und einem nicht resorbierbaren Faden, der durch das Band gestochen wurde und an der Schraube verknotet wurde, hergestellt.

Nach kurzer Zeit berichtete der Patient über eine subjektive Besserung der Tagesmüdigkeit.

### Beispiel 3

Der Patient hatte eine mehrjährige Krankengeschichte hinter sich, die durch ausgeprägte Tagesmüdigkeit, Einschlafneigung am Steuer und während der Arbeit und Leistungsschwäche gekennzeichnet war. Zusätzlich zur konservativen Therapie mit nCPAP war eine Operation des Weichgaumens ausgeführt worden, jedoch ohne Erfolg. Es wurde dasselbe Operationsverfahren wie in Beispiel 2 vorgenommen. Das Silikonband wurde am Unterkiefer mit einer Titanplatte festgeklemmt. Es zeigten sich postoperativ keine Komplikationen. Am dritten Tag nach der Operation wurde der Patient entlassen und berichtete wenige Zeit später über eine subjektive Besserung der Tagesmüdigkeit. Da keine vollständige Restitutio erfolgt war, war ein Nachspannen des Fadens geboten.

Bei keinem der Patienten zeigte sich eine unerwünschte Wirkung des Verfahrens. Die postoperativen Schmerzen erwiesen sich als erheblich geringer als beim Repose-Verfahren aus dem Stand der Technik. Als vorteilhaft erwiesen sich die anatomisch günstig konfigurierte Ankerplatte und die leicht justierbare Fadenspannung, die die Ergebnisse weiter verbesserte. Die durch eine Implantation des erfindungsgemäßen Systems erzielten Behandlungserfolge erwiesen sich in den Behandlungsergebnissen mit den Operationsverfahren aus dem Stand der Technik (Repose, Hyoidsuspension, UPPP, Somnoplastie) überlegen.

## Patentansprüche

1. Vorrichtung zur submentalen Implantation in einen Zungenkörper umfassend mindestens ein elastisches Element, umfassend einen Anker und ein Band, wobei das Band an einer oder mehreren Seiten Vertiefungen, Erhöhungen oder Durchtrittslöcher besitzt, sodass das elastische Element die Justierung der erforderlichen Kraft des Ankers gegen den Zungenkörper in die gewünschte Richtung erlaubt.

2. Vorrichtung nach Anspruch 1, **dadurch gekennzeichnet, dass** zur Festlegung des Bandes ein Widerlager vorgesehen ist.

3. Vorrichtung nach einem der Ansprüche 1 oder 2, **dadurch gekennzeichnet, dass** das elastische Element in Form einer Spange oder eines Rings vorliegt.

4. Vorrichtung nach einem der Ansprüche 1 bis 3, **dadurch gekennzeichnet, dass** das Band als Polyeder, Rotationskörper oder Teil eines Rotationskörpers ausgeführt ist.

5. Vorrichtung nach Anspruch 4, **dadurch gekennzeichnet, dass** das Band an den Kanten abgerundet ist.

6. Vorrichtung nach einem der Ansprüche 1 bis 5, **dadurch gekennzeichnet, dass** der Anker eine runde, elliptische oder ovale Form besitzt.

7. Vorrichtung nach einem der Ansprüche 1 bis 6, **dadurch gekennzeichnet, dass** der Anker Perforationen aufweist.

8. Vorrichtung nach einem der Ansprüche 1 bis 7, **dadurch gekennzeichnet, dass** der Anker mit kontrastgebendem Material dotiert ist.

9. Vorrichtung nach einem der Ansprüche 1 bis 8, **dadurch gekennzeichnet, dass** der Anker zum peripheren Bereich eine abnehmende Materialstärke besitzt.

10. Vorrichtung nach einem der Ansprüche 1 bis 9, **dadurch gekennzeichnet, dass** der Anker gerade ist.

11. Vorrichtung nach einem der Ansprüche 1 bis 9, **dadurch gekennzeichnet, dass** der Anker gekrümmt ist.

12. Vorrichtung nach einem der Ansprüche 1 bis 11, **dadurch gekennzeichnet, dass** der Anker in geschlossenem Zustand implantierbar und regenschirmartig öffenbar ist.

13. Vorrichtung nach einem der Ansprüche 2 bis 12, **dadurch gekennzeichnet, dass** das Widerlager eine Schraube, eine Klemmung, eine elektromechanische Verriegelung oder eine Kombination aus Sockel und Verschlusskappe ist.

14. Vorrichtung nach einem der Ansprüche 1 bis 13, **dadurch gekennzeichnet, dass** das Band des elastischen Elements ein Band mit Fischschuppenstruktur ist.

15. Vorrichtung nach einem der Ansprüche 1 bis 14, **dadurch gekennzeichnet, dass** das Band am Anker lösbar befestigt ist.

16. Vorrichtung nach einem der Ansprüche 1 bis 15 zur Verwendung in einem Verfahren zur Behandlung von Störungen, die durch fehlerhafte oder mangelnde Positionierung eines menschlichen oder tierischen Zungenkörpers hervorgerufen werden.

17. Vorrichtung nach Anspruch 16 zur Verwendung in einem Verfahren zur Behandlung von Atmungsstörungen, schlafbezogenen Atmungsstörungen, obstruktivem Schnarchen und Glossoptose.

## Claims

1. A device for submental implantation into a tongue body, comprising at least one elastic element comprising an anchor and a band, wherein the band has recesses, raised parts or passage apertures at one or several sides so that the elastic element allows the adjustment of the necessary force of the anchor against the tongue body in the desired direction.

2. The device according to claim 1, **characterized in that** an abutment is provided for fixing the band.

3. The device according to any one of claims 1 or 2, **characterize**d in t h a t the elastic element is present in form of a clip or a ring.

4. The device according to any one of claims 1 to 3, **characterized in that** the band is implemented as a polyhedron, rotational body or part of a rotational body.

5. The device according to claim 4, **characterized in that** the band is rounded at the edges.

6. The device according to any one of claims 1 to 5, **characterized in that** the anchor has a round, elliptic or oval form.

7. The device according to any one of claims 1 to 6, **characterized in that** the anchor has perforations.

8. The device according to any one of claims 1 to 7, **characterized in that** the anchor is doped with a contrast-giving material.

9. The device according to any one of claims 1 to 8, **characterized in that** the anchor has a material thickness that is decreasing towards the peripheral region.

10. The device according to any one of claims 1 to 9, **characterized in that** the anchor is straight.

11. The device according to any one of claims 1 to 9, **characterized in that** the anchor is bent.

12. The device according to any one of claims 1 to 11, **characterized in that** the anchor is implantable in a closed state and can be opened like an umbrella.

13. The device according to any one of claims 2 to 12, **characterized in that** the abutment is a screw, a clamping, an electromechanical locking or a combination of base and locking cap.

14. The device according to any one of claims 1 to 13, **characterized in that** the band of the elastic element is a band having a fish scale structure.

15. The device according to any one of claims 1 to 14, **characterized in that** the band is removably attached to the anchor.

16. The device according to any one of claims 1 to 15 for use in a method for treatment of disorders that are caused by a deficient or inadequate positioning of a human or animal tongue body.

17. The device according to claim 16 for use in a method for treatment of breathing disorders, sleep-related breathing disorders, obstructive snoring and glossoptosis.

## Revendications

1. Dispositif d'implantation sous-mentale dans le corps de la langue, comprenant au moins un élément élastique comprenant une ancre et une sangle, où la sangle est pourvue d'évidements, de bosses ou de trous de passage sur une ou plusieurs côtés, de manière à permettre à l'élément élastique d'ajuster la force exigée pour l'ancre contre le corps de la langue dans la direction souhaitée.

2. Dispositif selon la revendication 1, **caractérisé en ce qu'**une butée est prévue pour la fixation de la sangle.

3. Dispositif selon la revendication 1 ou la revendication 2, **caractérisé en ce que** l'élément élastique se présente sous la forme d'une boucle ou d'un anneau.

4. Dispositif selon l'une des revendications 1 à 3, **caractérisé en ce que** la sangle est réalisée comme polyèdre, corps de révolution, ou comme une partie d'un corps de révolution.

5. Dispositif selon la revendication 4, **caractérisé en ce que** la sangle est arrondie sur ses bords.

6. Dispositif selon l'une des revendications 1 à 5, **caractérisé en ce que** l'ancre présente une forme ronde, elliptique ou ovale.

7. Dispositif selon l'une des revendications 1 à 6, **caractérisé en ce que** l'ancre est pourvue de perforations.

8. Dispositif selon l'une des revendications 1 à 7, **caractérisé en ce qu'**une substance contrastante est ajoutée à l'ancre.

9. Dispositif selon l'une des revendications 1 à 8, **caractérisé en ce que** l'ancre présente une épaisseur de matériau décroissante vers sa périphérie.

10. Dispositif selon l'une des revendications 1 à 9, **caractérisé en ce que** l'ancre est droite.

11. Dispositif selon l'une des revendications 1 à 9, **caractérisé en ce que** l'ancre est incurvée.

12. Dispositif selon l'une des revendications 1 à 11, **caractérisé en ce que** l'ancre est implantable en état de fermeture et peut être ouverte à la façon d'un parapluie.

13. Dispositif selon l'une des revendications 2 à 12, **caractérisé en ce que** la butée est une vis, un tendeur, un verrou électromécanique ou une combinaison d'un socle et d'un bouchon.

14. Dispositif selon l'une des revendications 1 à 13, **caractérisé en ce que** la sangle de l'élément élastique est une sangle à structure en écailles de poisson.

15. Dispositif selon l'une des revendications 1 à 14, **caractérisé en ce que** la sangle est fixée de manière amovible sur l'ancré.

16. Dispositif selon l'une des revendications 1 à 15, destiné à être utilisé dans le cadre d'un processus de traitement de troubles dus à une position défectueuse ou insatisfaisante d'un corps de langue humain ou animal.

17. Dispositif selon la revendication 16, destiné à être utilisé dans le cadre d'un processus de traitement de troubles respiratoires, de troubles respiratoires liés au sommeil, de ronflements obstructifs et de la glossoptose.
